# FASCICULE DE BREVET EUROPEEN

(11) **EP 0 021 973 B1**
(45) Date de publication et mention de la délivrance du brevet: **08.12.1982**
(21) Numéro de dépôt: 80400874.6
(22) Date de dépôt: 17.06.1980
(51) Int. Cl.: A61K 31/445, C07D 401/04

(54) **Aminopipéridines anorexigènes, procédé pour leur préparation et médicaments les contenant**
Appetithemmende Aminopiperidine, Verfahren zu ihrer Herstellung und sie enthaltende Arzneimittel
Anorectic amino piperidines, process for their preparation and medicaments containing them

(30) Priorité: 21.06.1979 FR 7915974
(43) Date de publication de la demande: 07.01.1981
(60) Demande divisionnaire: 81107653.8
(73) Titulaire: SANOFI S.A., 75008 Paris (FR)
(72) Inventeur: Nisato, Dino, Pavia (IT); Carminati, Paolo, Milano (IT)
(74) Mandataire: de Haas, Michel

## Description

La présente invention concerne de nouvelles aminopipéridines ayant une activité anorexigène, un procédé pour leur préparation, ainsi que des médicaments contenant ces aminopipéridines en tant que principes actifs.

Plus particulièrement, l'invention se réfère à des 4-amino-1-(2-pyridyl)pipéridines de formule générale 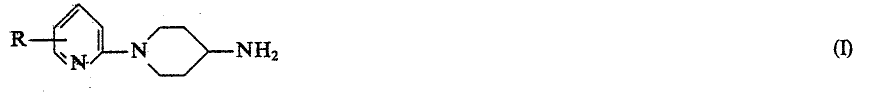 dans laquelle R représente de l'hydrogène, un halogène, un groupe méthyle, un groupe trifluorométhyle, un groupe alkoxy inférieur, un groupe trifluorométhoxy, un groupe 2,2,2-trifluoro- éthoxy, un groupe alkylthio inférieur, un groupe trifluorométhylthio, un groupe phénoxy, un groupe phénoxy substitué par un halogène, un groupe trifluorométhyle, un alkyle inférieur, un alkoxy inférieur, un alkyilthio-inférieur ou un groupe cyano, un groupe phénylthio un groupe phénylthio substitué dans le noyau phényle par un atome d'halogène, un groupe trifluorométhyle, un alkyle inférieur, un alkoxy inférieur, un alkylthio inférieur ou un groupe cyano; ainsi qu'à leurs sels pharmaceutiquement acceptables.

Les termes »alkyle inférieur«, »alkoxy inférieure et »alkylthio inférieur«, tels qu'utilisés ici, désignent des groupes contenant le résidu d'un hydrocarbure aliphatique saturé contenant 1 à 3 atomes de carbone, à savoir méthyle, éthyle, propyle et isopropyle.

Le terme »halogène« indique l'un des quatre halogènes communs, le fluor, le chlore et le brome étant particulièrement préférés.

Les sels pharmaceutiquement acceptables des 4-amino-1-(2-pyridyl)pipéridines de l'invention comprennent les sels non toxiques dérivés d'acides minéraux ou organiques, salifiant une ou bien les deux fonctions basiques présentes dans la molécule des composés de formule 1 ci-dessus, tels que les chlorhydrates, les bromhydrates, les succinates, les tartrates; les citrates, les fumarates, les maléates, les pamoates, les napsylates, les mésylates, les tosylates.

Le brevet DE-C-738 495 décrit un procédé pour la préparation de 4-aminopipéridines ayant la formule générale suivante 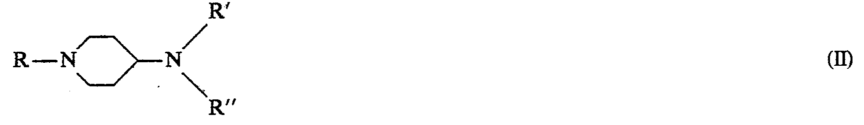 dans laquelle R représente un groupe aliphatique, aromatique, alicyclique ou hétérocyclique et R' et R" représentent de l'hydrogène ou des groupes alkyle.

Les composés de formule Il sont censés servir à des buts thérapeutiques; ils montrent des propriétés spasmolytiques. Ledit brevet décrit la 8'-[4-(diméthylamino)pipéridyl]quinoléine, mais il ne cite aucune aminopipéridine directement liée à un noyau hétérocycle, en particulier à une pyridine.

'On a maintenant trouvé que les nouvelles aminopipéridines de formule I possèdent une activité anorexigène remarquable et qu'elles peuvent donc être utilisées pour le traitement de l'obésité.

On a aussi trouvé que l'activité anorexigène des nouvelles aminopipéridines de formule I est sélective et ne comporte donc pas d'effects secondaires au niveau du système nerveux central. En particulier, les nouveaux composés de l'invention agissent sur le système sérotoninergique central sans effet sur le système dopaminergique et avec une action modérée sur le système noradrénergique.

Selon l'invention, les nouvelles 4-amino-1-(2-pyridyl)pipéridines sont préparées en faisant réagir une 2-halopyridine de formule 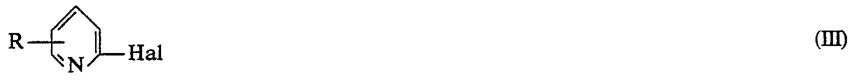 dans laquelle R a la signification donnée ci-dessus et Hal représente un atome d'halogène, avec une aminopipéridine bloquée de formule 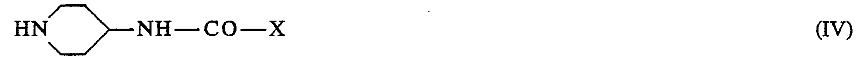 dans laquelle X représente un atome d'hydrogène ou un groupe alkyle inférieur et en hydrolysant ensuite la 4-amino-1-(2-pyridyl)pipéridine N-substituée ainsi obtenue de formule 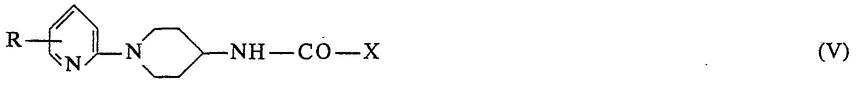 dans laquelle R et X ont les significations données ci-dessus, dans des conditions acides ou basiques.

La 4-acétamidopipéridine (formule IV, X = méthyle) représente la 4-aminopipéridine bloquée préférée.

La réaction de la 2-halopyridine de formule III avec-la 4-aminopipéridine bloquée est conduite dans un solvant organique tel qu'un alcool, n-butanol, n-pentanol ou n-hexanol de préférence, diméthylformamide, diméthylsulfoxyde, sulfolane, acétonitrile, pyridine et similaires, à une température entre 50 et 200° C, en présence d'un agent de condensation alcalin, tel qu'un hydroxyde, un carbonate ou un bicarbonate alcalin. Après 20 à 120 h, la réaction est complète et la 4-amino-1-(2-pyridyl)pipéridine N-substituée de formule V ainsi obtenue est isolée selon les techniques habituelles.

Pour accélérer la réaction, il peut être opportun d'opérer en présence d'un catalyseur, par exemple en présence de cuivre.

Le déblocage du groupe amino substitué est effectué suivant les techniques d'hydrolyse acide ou basique bien connues.

L'hydrolyse acide est effectuée, par exemple, en chauffant la 4-amino-1-(2-pyridyl)pipéridine bloquée de formule V dans une solution aqueuse d'un acide minéral ou organique acide chlorhydrique de préférence, et la 4-amino-1-(2-pyridyl)pipéridine de formule I est isolée selon des techniques conventionnelles.

L'hydrolyse en milieu basique est effectuée par traitement du composé de formule V avec une base minérale, hydroxyde de sodium de préférence. A la fin de l'hydrolyser la 4-amino-1-(2-pyridyi)pipéri- dine est isolée et/ou transformée dans ses sels d'addition par traitement avec une solution aqueuse hydroalcoolique ou dans un solvant organique de l'acide salifiant.

Les 4-aminopipéridines bloquées de formule IV employées comme produits de départ peuvent être préparées. selon des méthodes connues en littérature, par exemple par réduction catalytique des 1-benzyl-4-aminopipéridines bloquées correspondantes ou par réduction des 4-aminopyridines bloquées correspondantes.

La 4-acétamidopipéridine, produit de départ préférentiel, est décrite dans le brevet US-A-3 124586.

Les nouvelles 4-amino-1-(2-pyridyl)pipéridines de l'invention et leurs sels montrent une activité anorexigène remarquable et sélective sans donner aucune action du type amphétaminique. La sélectivité de leur action est démontrée par le manque d'activités pharmacologiques. secondaires, telles que l'activité sédative ou excitante et l'action inhibitrice de l'activité locomotrice.

Les composés de l'invention ne montrent pas d'action spasmolytique ni d'effets secondaires cardiovasculaires indésirables.

Les composés de formule I ci-dessus, dans laquelle le substituant R est dans la position 6 du noyau de la pyridine, sont particulièrement préféres.

Par rapport à leur degré d'activité, les 4-amino-1-(2-pyridyl)pipéridines de l'invention sont peu toxiques et présentent un bon index thérapeutique. Dans le tableau 1 ci-dessous, la toxicité aiguë est exprimée sous forme de »zone de mortalité«. On a utilisé 6 lots de souris mâles de 20 g environ, auxquelles les composés de l'invention, dissous ou suspendus dans de la carboxyméthylcellulose, ont été injectés par voie intrapéritonéale à moins quatre doses pour chaque lot d'animaux. Les animaux ont été observés pendant 48 h après le traitement, et le nombre des animaux décédés ainsi que le type de mort ont été enregistrés. La »zone de mortalité« est représentée par les doses auxquelles on observe la mortalité la plus basse (au moins un mort) et la plus élevée.

L'activité anorexigène a été évaluée par la méthode de la prise de nourriture chez le rat. On a utilisé des rats femelles de 200 g entraînés pendant 10 jours à manger durant une période de 4 h et sélectionnes au 8e jour. Au bout du 10e jour, les animaux randomisés ont été divisés en un »groupe contrôle«, traité par le seul véhicule, et en plusieurs »groupes traités«. Le traitement a été effectué par voie intrapéritonéale 30 min avant la présentation de la nourriture et, ensuite, on a mesuré la quantité de nourriture consommée au cours de la première heure. Les données reportées dans le tableau I ci-dessous indiquent le pourcentage d'inhibition de la prise de nourriture des animaux traités par des composés représentatifs de l'invention par rapport aux contrôles.

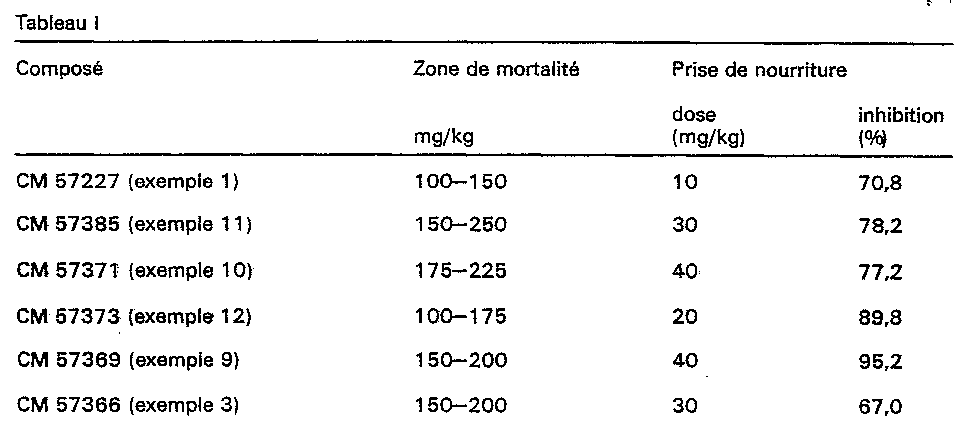

Du tableau 1 ci-dessus, il ressort que les composés de l'invention, à des doses entre environ 1/10 et 1/5 de la dose minimale toxique chez la souris, montrent une diminution remarquable de la prise de nourriture.

L'activité anorexigène par voie orale d'un produit représentatif de l'invention, la 4-amino-1-(6-chlo- ro-2-pyridyl)pipéridine sous forme de monochlorhydrate (CM 57 227, composé de l'exemple 1), a été testée selon la méthode de la prise de nourriture décrite ci-dessus; comme produits de référence, on a utilisé un anorexigène très connu, la fenfluramine, ainsi que la 1-(6-chloro-2-pyridyl)pipérazine sous forme de monochlorhydrate, décrite dans le brevet US-A-4 078 063, et la 1-(6-chloro-2-pyrazinyl)pipé- razine sous forme de monochlorhydrate, décrite dans le brevet GB-A-1 492 528.

Les produits ont été évalués aussi en fonction de leur action sur l'activité locomotrice chez le rat. A ce but, on a utilisé des rats femelles de 200 g environ, auxquels les composés ont été administrés par voie orale. Au bout de 30 min, les animaux traités ont été mis dans une cage (80 x 80 cm) munie d'une cellule photo-électrique pour la mesure des mouvements spontanés et le nombre d'impulsions transmises à la cellule pendant 10 min a été enregistré. L'inhibition de l'activité locomotrice de chaque substance (6 à 10 animaux pro dose) a été exprimée en pourcentage par rapport aux contrôles.

Dans le tableau Il ci-dessous sont reportés

―la toxicité aiguë, exprimée comme DL₅₀ chez le rat par voie orale (donnée A),
-l'activité anorexigène, exprimée comme dose orale inhibant de 50% la prise de nourriture (DE₅₀, donnée B),
-l'action sur l'activité locomotrice, exprimée comme dose orale inhibant de 50% la motilité spontanée, à savoir le nombre d'impulsions transmises à la cellule photo-électrique pendant les 10 min (DI₅₀, donnée C),
-le rapport entre la toxicité aiguë et l'activité anorexigène, qui exprime l'index thérapeutique lié à la toxicité aiguë (donnée A/B),
-le rapport entre l'action sur l'activité locomotrice et l'activité anorexigène, qui exprime l'index thérapeutique lié à l'inhibition de l'activité locomotrice, signe d'un effet secondaire du type sédatif ou dépressif (donnée C/B).

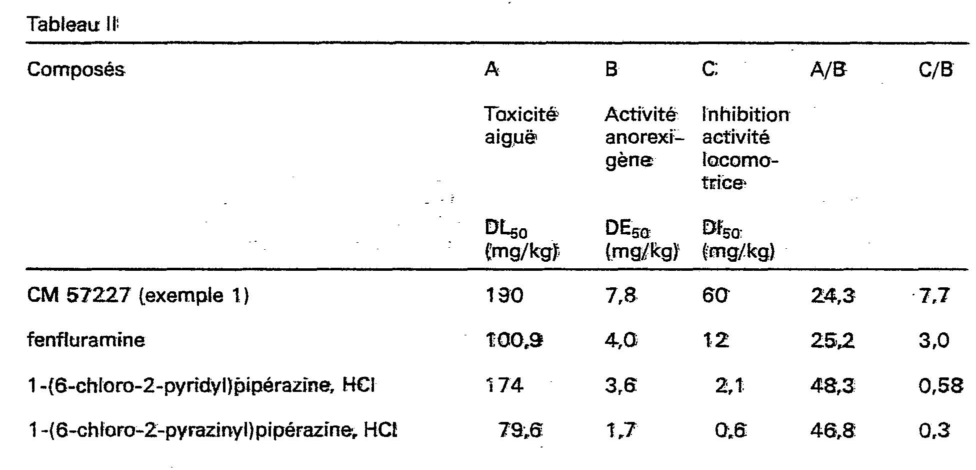

Du tableau II ci-dessus, il ressort que le produit représentatif de l'invention possède la moindre toxicité, tout en conservant un haut degré d'activité anorexigène, et ne montre une inhibition de l'activité locomotrice qu'à des doses environ 8 fois supérieures à la DEso anorexigène: Des composés de référence; la 1-(6-chloro-2-pyridyl)pipérazine et la 1-(6-chloro-2-pyrazinyl)piperazine qui, dans le test employé, sont très actives comme anorexigènes, inhibent l'activité locomotrice a des doses même inférieures à la DE₅₀ anorexigène, tandis que la fenfluramine, dont l'index thérapeutique A/B est presque égal à celui de la 4-amino-1-(6-chloro-2 pyridyl)pipéridine de l'invention, a une action inhibitricede l'activité locomotrice bien supérieure.

Sur le plan biochimique; les nouvelles 4-amino-1-(2-pyridyl)pipéridines de formule 1 et leurs sels se révèlent des inhibiteurs remarquable de l'uptake de la sérotonine ayant aussi un effet inhibiteur modéré de l'uptake de la noradrénaline et pas d'action inhibitrice de l'uptake de la dopamine; ce qui comporte une activité sélective sur les amines cérébrales et des effets secondaires réduits.

En particulier, les composés de l'invention, in vivo, ne provoquent pas de déplétion de sérotonine au niveau central. Le tableau III ci-dessous résume les taux cérébraux de sérotonine après administration intrapéritonéale chez le rat d'un composé représentatif de l'invention, la 4-amino-1-(6-chloro-2-pyri- dyl)pipéridine sous forme de monochlorhydrate (CM-57 227, produit de l'exemple 1). La fenfluramine a été utilisée produit de référence.

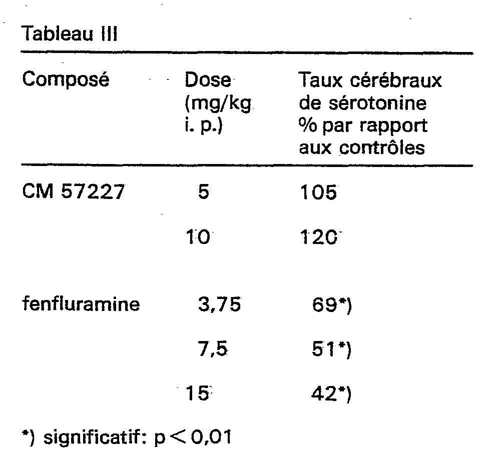

### Les animaux ont été traités 60 min avant le sacrifice.

De ce tableau-III, il ressort que le produit de l'invention, à des doses anorexigènes, ne modifie pas les taux cérébraux de sérotonine, tandis que la fenfluramine, à des doses équivalentes, provoque une réduction significative de sérotonine cérébrale déjà décrite en littérature (Arch. lntern. Pharmacodyn. Ther., 1967,170,276).

Etant donné qu'une déplétion durable de sérotonine peut être considérée comme un signe de neurotoxicité potentielle (C. D. Morgan et ai., Life Sci., Part 1,11, 83,1972), il y a une moindre possibilité que l'usage prolongé du composé de l'invention provoque des effets secondaires au niveau central.

En raison de leur bonne activité anorexigène, de leur basse toxicité par rapport au degré d'activité et de l'absence d'effets secondaires majeurs, les composés de l'invention peuvent être utilisés comme agents anorexigènes à des doses de 0,01 à 10 mg/kg par jour. Ils peuvent être administrés par voie orale, sublinguale, subcutanée, intramusculaire, intraveineuse, transdermique ou rectale dans des compositions pharmaceutiques contenant 0,5 à 250 mg de principe actif par chaque unité de dosage, en mélange avec les excipients conventionnels. La voie d'administration préférée est la voie orale, sous forme de comprimés, comprimés retard, dragées, sachet, suspensions, sirops.

Les exemples suivants illustrent l'invention sans toutefois la limiter.

### Exemple 1

On chauffe au reflux sous agitation pendant 40 h une suspension de 175,5 g de 4-acétamidopipéridine, 269,7 g de carbonate de potassium et 182,5 g de 2,6-dichloropyridine dans 1600 ml d'alcool n-pentylique. On refroidit le mélange réactionnel, on élimine le solvant sous pression réduite et on reprend le résidu dans 1000 ml d'eau. On extrait le produit solide dans 2000 ml de chlorure de méthylène, on lave la phase organique avec de l'eau, on la sèche sur du sulfate de sodium anhydre et on l'évapore à sec. On reprend le résidu dans 1200 ml d'éther diisopropylique, on agite pendant 30 min, on filtre et on lave avec de l'éther diisopropylique. On obtient 246 g de 4-acétamido-1 -(6-chloro-2-pyri- dyl) pipéridine, F. 153-155°C.

On chauffe au reflux pendant 8 h une solution de 245 g de 4-acétamido-1-(6-chloro-2-pyridyl)pipéri- dine dans 1220 ml d'acide chlorhydrique 6N. On refroidit le mélange réactionnel et on le concentre sous pression réduite. On traite le résidu avec de l'hydroxyde de sodium 4N jusqu'à un pH nettement basique et on extrait l'huile qui se sépare avec de l'éther diéthylique. On lave la phase organique avec de l'eau, on la sèche sur du sulfate de sodium anhydre et on l'évapore à sec. On verse, à 0° C, le résidu buileux dans 350 ml d'acide chlorhydrique concentré, on décolore la solution ainsi obtenue avec du charbon et on ajuste à 5 le pH de la solution limpide. On filtre le solide qui précipite, on le lave trois fois avec 50 ml d'eau et on le cristallise dans de l'eau en refroidissant à 3―4°C. On sèche le produit ainsi obtenu sur P₂O₅ à 50° C sous pression réduite. On obtient 200 g de monochlorhydrate de 4-amino-1-(6-chloro-2-pyridyl)pipéridine (CM 57 227) ayant un point de fusion d'environ 300°C avec décomposition.

### Exemple2

Suivant le mode opératoire décrit à l'exemple 1, par réaction de la 4-acétamidopipéridine avec la 2,3-dichloropyridine, on obtient, avec un rendement de 70%, la 4-acétamido-2-(3-chloropyridyl)pipéri- dine qui, après cristallisation dans de l'acétate d'éthyle, fond à 118―120° C. Par hydrolyse du produit ainsi obtenu avec de l'acide chlorhydrique 6N, on obtient le monochlorhydrate de 4-amino-1 -(3-chloro- pyridyl)pipéridine (CM 57375) qui est cristallisé dans de l'éthanol à 90%; F. 258-260°C avec décomposition, Rendement: 67%.

### Exemple 3

On chauffe sous agitation à 130°C, pendant 50 h, un mélange de 0,088 mole de 4-acétamidopipéridine, 15 g de carbonate de potassium, 0,088 mole de 2-chloro-6-méthoxypyridine dans 100 ml de diméthylsulfoxyde, puis on refroidit, in verse le mélange dans de l'eau et on extrait la suspension ainsi obtenue avec de l'éther diéthylique. On extrait la phase aqueuse avec du chlorure de méthylène, on lave la phase organique avec de l'eau, on la sèche sur du sulfate de sodium anhydre et on l'évapore à sec. On obtient ainsi la 4-acétamido-1-(6-méthoxy-2-pyridyl)pipéridine qui, après cristallisation dans de l'éthanol à 95%, fond à 125 ― 127° C. Rendement: 43% de la théorie.

On chauffe au reflux pendant 20 h une solution de 10 g de 4-acétamido-1 -(6-méthoxy-2-pyridyl)pipé- ridine, 44 g d'hydroxyde de sodium en pastilles, 44 ml d'eau et 200 ml d'éthylèneglycol, puis on refroidit, on verse le mélange réactionnel dans de l'eau, on sature la solution ainsi obtenue par du chlorure de sodium et l'on extrait par de l'éther diéthylique. On lave la phase organique avec une solution aqueuse saturée de chlorure de sodium, on la sèche sur du sulfate de sodium et on la concentre jusqu'à siccité. On dissout l'huile résiduelle dans 160 ml d'isopropanol et on traite la solution ainsi obtenue avec une solution d'acide chlorhydrique en isopropanol. On filtre le solide qui se sépare, on le lave avec de l'isopropanol et on le recristallise dans de l'éthanol. On obtient 8,5 g de monochlorhydrate de 4-amino-1-{6-méthoxy-2-pyridyl)pipéridine (CM 57366); F. 225-235°C (avec décomposition). Rendement: 87%.

### Exemple4

On chauffe sous agition, pendant 50 h, à 130°C, un mélange de 12,5 g de 4-acétamidopipéridine, 15 g de carbonate de potassium, 10 g de 2-chloropyridine dans 100 ml de diméthylsulfoxyde, puis on le refroidit, on le verse dans l'eau et on extrait la suspension ainsi obtenue avec de l'éther diéthylique. On extrait la phase aqueuse avec du chlorure de méthylène, on lave la phase organique avec de l'eau, on la sèche sur du sulfate de sodium anhydre et on l'évapore à sec. On obtient ainsi la 4-acétamido-1-(2-pyridyi)pipéridine qui, après cristallisation dans de l'isopropanol et recristallisation dans de l'acétate d'éthyle, fond à 165-168° C. Rendement: 4,7 g (24,5% de la théorie).

Par hydrolyse de la 4-acétamido-1-(2-pyridyl)pipéridine ainsi obtenue en présence d'acide chlorhydrique 6N suivant la technique opératoire décrite à l'exemple 1, on obtient le dichlorhydrate de 4-amino-1-(2-pyridyl)pipéridine (CM 57359); F.>260°C (avec décomposition). Rendement: 72% de la théorie.

### Exemple 5

On chauffe à 150°C sous agitation, pendant 40h, un mélange de 0,116 mole de 2-chloro-6-phénoxypyridine, 21,5 g de carbonate de potassium anhydre, 0,151 mole de 4-acétamidopyridine et 0,118 mole de cuivre en poudre dans 100 ml de sulfolane. On distille le sulfolane, on reprend le résidu dans de l'eau, on traite la suspension avec de l'eau et on l'extrait dans du chlorure de méthylène. On lave la phase organique avec de l'eau, on la sèche et on la concentre jusqu'à siccité. Par cristallisation du résidu dans de l'éther diisopropylique, on obtient la 4-acétamido-2-(6-phénoxy-2-py- ridyl)pipéridine; F. 104-106° C. Rendement: 30% de la théorie.

En hydrolysant la 4-acétamido-2-(6-phénoxy-2-pyridyl)pipéridine ainsi obtenue avec de l'hydroxyde de sodium, en versant le mélange réactionnel dans de l'eau et en le traitant avec de l'acide chlorhydrique comme décrit à l'exemple 3, on obtient le monochlorhydrate de 4-amino-2-(6-phénoxy-2-pyridyl)pipéridine (CM 57 380); F.195-198° C: Rendement: 25% de la théorie.

### Exemples 6 à 8

Suivant le mode opératoire décrit à l'exemple 5, par réaction de la 4-acétamidopipéridine avec la 2-chloro-4-méthylpyridine, la 2-chloro-6-(méthylthio)pyridine et la 2-chloro-6-(isopropylthio)pyridine (E. 130°C/16 mm Hg, préparée par réaction de la 2,6-dichloropyridine avec l'isopropylmercaptan, sel de Na), on obtient les 4-acétamido-1-(2-pyridyl)pipéridines correspondantes, qui, par hydrolyse avec de l'hydroxyde de sodium, donnent les 4-amino-(2-pyridyl)pipéridines. Les caractéristiques physico-chimiques et les rendements des réactions sont résumés dans le tableau IV suivant.

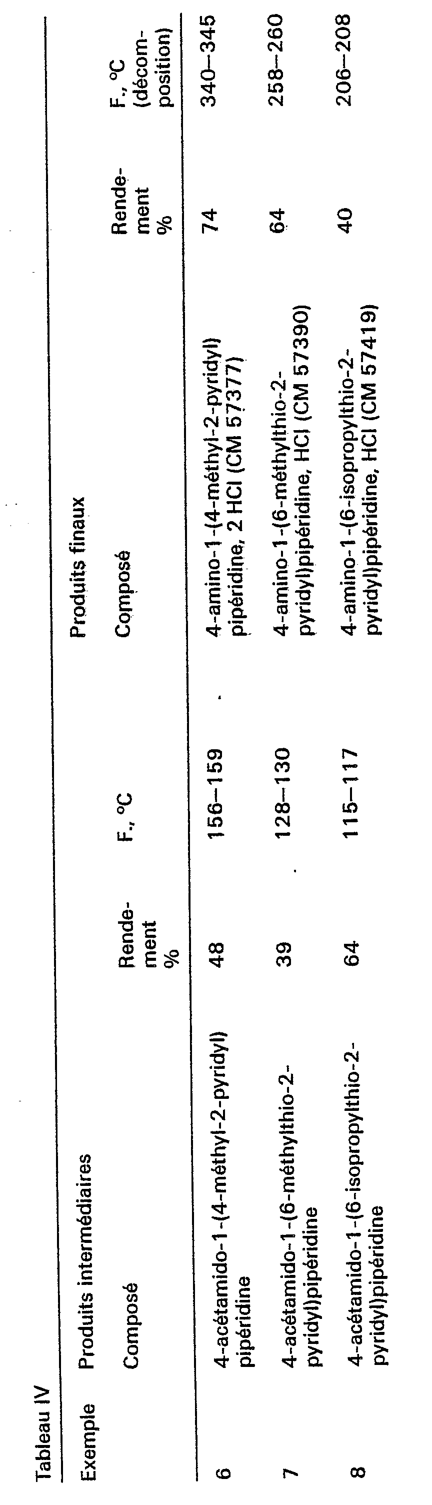

### Exemple 9

On chauffe à 150° C sous agitation, pendant 50 h, un mélange de 9,5 g de 2-chloro-6-(phénylthio)pyridine (E. 112-116°C/0,01 mm Hg, préparée par réaction de la 2,6-dichloropyridine avec le sel sodique du thiophénol), de 7,8 g de carbonate de potassium et de 7,6 g de 4-acétamidopyridine dans 75 ml de sulfotane, puis on concentre le sulfolane à 1/5 de son volume, on verse le mélange dans 100 ml d'eau et on extrait 4 fois avec 150 mt d'éther diéthylique. On lave la phase organique avec de l'eau, on la sèche sur du sulfate de sodium anhydre, on la concentre jusqu'à siccité et on cristallise le résidu dans 40 ml d'acétate d'éthyle. On obtient ainsi 6,8 g de 4-acétamido-1-(6-phénlthio-2-pyridyl)pipéridine; F-110-112°C.

En hydrolysant la 4-acétamide-2-(6-phénylthio-2-pyridyl)pipéridine ainsi obtenue avec. de l'hydroxyde de sodium, en versant le mélange réactionnel dans de l'eau et en le traitant avec de l'acide chlorhydrique 6N comme décrit à l'exemple 3, on obtient ie monochlorhydrate de 4-amino-1 -(6-phénylthio-2-pyridyl)pipéridine (CM 57 369) qui, après cristallisation dans le l'éthanol, fond à 245-247°C avec décomposition. Rendement: 57,5%.

### Exemple 10

On chauffe à 150°C, sous agitation, pendant 24 ₕ 0,042 mole de 2-chloro-6-méthylpyridine, 0,056 mole de carbonate de potassium anhydre et 0,052 mole de 4-acétamidopyridine dans 75 mt de sulfolane, puis on concentre le sulfolane jusqu'à 1/5 de son volume, on verse le mélange dans 100 mi d'eau on l'extrait 4 fois avec 150 ml d'éther diéthylique. On lave la phase organique avec de l'eau, on la sèche sur du sulfate de sodium anhydre, on la concentre jusqu'à siccité et on cristallise le résidu dans 40 ml d'acétate d'éthyle. On obtient ainsi la 4-acétamido-1-(6-méthyl-2-pyridyl)pipéridine; F. 145-147°C. Rendement: 28%.

Par hydrolyse de la 4-acétamido-1-(6-méthyl-2-pyridyl)pipéridine ainsi obtenue en présence d'acide chlorhydrique 6N suivant la technique opératoire décrite à l'exemple 1, on obtient le dichlorhydrate de 4-amino-1-(6-méthyl-2-pyridyl)pipéridine (CM 57371) qui, cristallisé dans de l'éthanol à 95%, fond à 308-310° C avec décomposition.

### Exemple 11

On chauffe à 100°C, sous agitation, pendant 50 h, un mélange de 19 g de 2-chloro-6-(2,2,2-trifluoro- éthoxy)pyridine (E. 80°C/16mmHg, préparée par réaction de la 2,6-dichloropyridine avec 2,2,2-trifluoroéthanolate de sodium), 14,87 g de 4-formamidopipéridine (F. 116―118° C, préparée par hydrogénation de la 4-formamido-1-benzylpipéridine en présence de palladium sur du charbon), 17,27 g de carbonate de potassium anhydre, 5,67 g de cuivre en poudre dans 110 ml de sulfolane, puis on distille le sulfolane, on reprend le résidu dans de l'eau et on extrait avec du chlorure de méthylène la suspension ainsi obtenue. On lave la phase organique avec de l'eau, on la sèche sur du sulfate de sodium anhydre et on l'évapore à sec. Le résidu, cristallisé d'abord dans un mélange eau/éthanol 84/16 en volume et après dans de l'éther diisopropylique, donne 9 g de 4-acétamido-1-[6-(2,2,2-trifluo- ro)éthoxy-2-pyridyl]pipéridine, F.92-95°C.

Par chauffage de la 4-acétamido-1 -[6-(2,2,2-trifluoro)-éthoxy-2-pyridyl]pipéridine au reflux pendant 3 h dans une solution 0,5 N d'acide chlorhydrique et opérant ensuite comme décrit à l'exemple 1, on obtient le monochlorhydrate de 4-amino-1-[6-(2,2,2-trifluoro)éthoxy-2-pyridyl]pipéridine (CM 57 385) qui, après cristallisation dans de l'éthanol, fond à 218 -225° C, avec décomposition. Rendement: 48,5% de la théorie.

### Exemple 12

On chauffe à 150° C, sous agitation, pendant20 h, un mélange de 0,042 mole de 2,6-dibromopyridine, 0,056 mole de carbonate de potassium anhydre et 0,052 mole de 4-acétamidopipéridine dans 75 ml de sulfolane, puis on concentre le sulfolane jusqu'à 1/5 de son volume, on verse le mélange réactionnel dans 100 ml d'eau et on extrait avec de l'éther diéthylique (4 x 150 ml). On lave la phase organique avec de l'eau, on la sèche sur du sulfate de sodium anhydre, on la concentre jusqu'à siccité et on cristallise le résidu dans 40 ml d'acétate d'éthyle. On obtient ainsi la 4-acétamido-1-(6-bromo-2-pyridyl)pipéri- dine; F. 158―160° C. Rendement: 56% de la théorie.

Par hydrolyse de la 4-acétamido-1-(6-bromo-2-pyridyt)pipéridine ainsi obtenue de l'acide chlorhydrique 6N suivant la technique opératoire décrite à l'exemple 1, on obtient le monochlorhydrate de 4-amino-1-(6-bromo-2-pyridyl)pipéridine (CM 57 373) qui, après cristallisation dans de l'éthanol à 95%, fond à 305―308° C avec décomposition. Rendement: 73% de la théorie.

### Exemple T3

On chauffe au reflux pendant 8 h une solution de 0,482 mole de 4-acétamido-1-(6-chloro-2-pyri- dyl)pipéridine dans 610 ml d'acide chlorhydrique 6N, puis on refroidit le mélange réactionnel et on le concentre sous pression.réduite. On traite le résidu avec de l'hydroxyde de sodium 4N jusqu'à un pH nettement basique et on extrait l'huile qui se sépare avec de l'éther diéthylique. On lave la phase organique avec de l'eau, on la sèche sur du sulfate de sodium anhydre et on l'évapore à sec. On cristatlise le résidu huileux par de l'éther diisopropytique. On obtient ainsi la 4-amino-1-(6-chloro-2-py- ridyl)pipéridine; F.36―38° C. Rendement: 72% de la théorie.

Une solution de 0,02 mole de 4-amino-1-(6-chloro-2-pyridyl)pipéridine en acétone est traitée par une solution de 0,02 mole d'acide fumarique en acétone. Le produit qui précipite est filtré, lavé avec de l'acétone et cristallisé dans de l'éthano à 95%. On obtient ainsi le fumarate de 4-amino-1-(6-chloro-2-pyridyl)pipéridine; F.238―240°C.(décomposition).

De la même façon, on obtient le maléate (F. 184―186°C), le tartrate (F. 205-208°C), le p-toluénesulfonate (F. 218-220°C), le succinate (F. 218-220°C avec décomposition), le 2-naphtalènesulfonate (F. 232―236° C avec décomposition) et le chlorhydrate cristallisé dans du méthane) identique au produit de l'exemple 1.

## Revendications

1. 4-amino-1-(2-pyridyl)pipéridines de formule générale 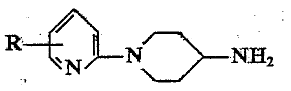 dans laquefle R représente de l'hydrogène, un halogène, un groupe méthyle, un groupe trifluorométhyle, un groupe alkoxy inférieur, un groupe trifiluorométhoxy, un groupe 2,2,2-trifluoro- éthoxy, un groupe alkylthio inférieur, un groupe trifluorométhylthio, un groupe phénoxy, un groupe phénoxy substitué dans le noyau phényle par un halogène, un groupe trifluorométhyle, un alkyle inférieur, un alkoxy inférieur, un alkylthio inférieur ou un groupe cyano, un groupe phénylthio, ou un groupe phénylthio substitué dans le noyau phényle par un halogène, un groupe trifluorométhyle, un alkyle inférieur, un alkoxy inférieur, un alkylthio inférieur ou un groupe cyano, et leurs sels pharmaceutiquement acceptables, les termes »alkyle inférieur«, »alkoxy inférieur et »alkylthio inférieure désignent des groupes contenant le résidu d'un hydrocarbure aliphatique saturé contenant 1 à 3 atomes de carbone.

2. 4-amino-1-(6-chloro-2 pyridyl)pipéridine et ses sels pharmaceutiquement acceptables.

3. Monochlorhydrate de 4-amino-1-(6-chloro-2-pyridyl)pipéridine:

4. Dichlorhydrate de 4-amino-1-[6-(2,2,2-trifluoro)éthoxy-2-pyridyl)pipéridine.

5. Dichlorhydrate de 4-amino-1-(6-méthyl-2-pyridyl)pipéridine.

6. Monochlorhydrate de 4-amino-1-(6-bromo-2-pyridyl)pipéridine.

7. Monochlorhydrate de 4-amino-1-(6-phénylthio-2-pyridyl)pipéridine.

8. Monochlorhydrate de 4-amino-1-(6-méthoxy-2-pyridyl)pipérdine.

9. Procédé pour la préparation de 4-amino-1-(2-pyridyl)pipéridines de formule 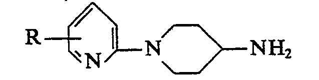 dans laquelle R représente de l'hydrogène, un halogène, un groupe méthyle, un groupe trifluorométhyle, un groupe alkoxy inférieur, un groupe trifluorométhoxy, un groupe 2,2,2-trifluoro- éthoxy, un groupe alkylthio inférieur, un groupe trifluorométhylthio, un groupe phénoxy, un groupe phénoxy substitué dans le noyau phényle par un halogène, un groupe trifluorométhyle, un alkyle inférieur, un alkoxy inférieur, un alkylthio inférieur ou un groupe cyano, un groupe phénylthio, ou un groupe phénylthio substitué dans le noyau phényle par un halogène, un groupe trifluorométhyle, un alkyle inférieur, un alkoxy inférieur, un alkylthio inférieur ou un groupe cyano, et de leurs sels pharmaceutiquement acceptables, caractérisé en ce qu'une 2-halopyridine de formule 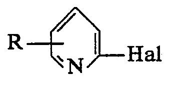 dans laquelle R a la signification donnée ci-dessus et Hal représente un atome d'halogène est traitée avec une 4-aminopipéridine bloquée de formule 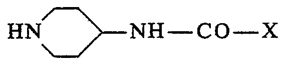 dans laquelle X représente un atome d'hydrogène ou un groupe alkyle inférieur, la 4-amino-1-(2-pyri- dyi)pipéridine N-substituée ainsi obtenue de formule 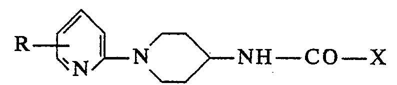 dans laquelle R et X ont les significations données ci-dessus, est hydrolysée dans des conditions acides ou basiques et le produit final est isolé et/ou transformé dans ses sels pharmaceutiquement acceptables.

10. Procédé suivant la revendication 9, caractérisé en ce que comme 4-aminopipéridine bloquée on utilise la 4-acétamidopipéridine.

11. Médicament renfermant, en tant que principe actif, un produit selon les revendications 1 à 8.

12. Médicament selon la revendication 11 sous forme de composition pharmaceutique contenant 0,5 à 250 mg de principe actif par chaque unité de dosage.

13. Médicament selon les revendications 11 et 12, caractérisé en ce qu'il est formulé pour l'administration orale.

## Patentansprüche

1. 4-Amino-1-(2-pyridyl)-piperidine der allgemeiner Formel 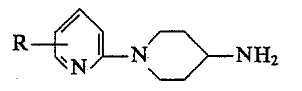 worin R für Wasserstoff, Halogen, Methyl, Trifluormethyl, Niedrigalkoxy, Trifluormethoxy, 2,2,2-Trifluoräthoxy, Niedrigalkylthio, Trifluormethylthio, Phenoxy, eine im Phenylkern durch Halogen, Trifluormethyl, Niedrigalkyl, Niedrigalkoxy, Niedrigalkylthio oder Cyano substituierte Phenoxygruppe, Phenylthio oder eine im Phenylkern durch Halogen, Trifluormethyl, Niedrigalkyl, Niedrigalkoxy, Niedrigalkylthio oder Cyano substituierte Phenylthiogruppe steht, und ihre pharmazeutisch akzeptablen Salze, wobei die Ausdrücke »Niedrigalkyl«, »Niedrigalkoxy« und »Niedrigalkylthio« Gruppen mit einem gesättigten aliphatischen Kohlenwasserstoffrest mit 1 bis 3 Kohlenstoffatomen bedeuten.

2. 4-Amino-1-(6-chlor-2-pyridyl)-piperidin und seine pharmazeutisch akzeptablen Salze.

3. 4-Amino-1 -(6-chlor-2-pyridyl)-piperidin-Monohydrochlorid.

4. 4-Amino-1-(6-(2,2,2-trifiluor)-äthoxy-2-pyridyl)-piperidin-Dihydrochlorid.

5. 4-Amino-1-(6-methyl-2-pyridyl)-piperidin-Dihydrochlorid.

6. 4-Amino-1-(6-brom-2-pyridyl)-piperidin-Monochiorid.

7. 4-Amino-1-(6-phenylthio-2-pyridyl)-piperidin-Monochlorid.

8. 4-Amino-1-(6-methoxy-2-pyridyl)-piperidin-Monohydrochlorid.

9. Verfahren zur Herstellung von 4-Amino-1-(2-pyridyl)-piperidinen der Formel 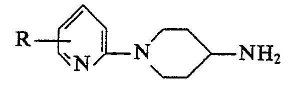 worin R für Wasserstoff, Halogen, Methyl, Trifluormethyl, Niedrigalkoxy, Trifluormethoxy, 2,2,2-Trifluoräthoxy, Niedrigalkylthio, Trifluormethylthio, Phenoxy, eine im Phenylkern durch Halogen, Trifluormethyl, Niedrigalkyl, Niedrigalkoxy, Niedrigalkylthio oder Cyano substituierte Phenoxygruppe, Phenylthio oder eine im Phenylkern durch Halogen, Trifluormethyl, Niedrigalkyl, Niedrigalkoxy, Niedrigalkylthio oder Cyano substituierte Phenylthiogruppe steht, und ihren pharmazeutisch akzeptablen Salzen, dadurch gekennzeichnet, daß 2-Halogenpyridin der Formel 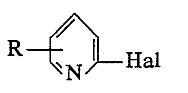 worin R obige Bedeutung-hat und Hai ein Halogenatom bedeutet, mit einem geschützten 4-Aminopiperidin der Formel 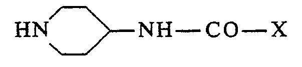 worin X Wasserstoff oder Niedrigalkyl bedeutet, behandelt wird, das so erhaltene N-substituierte 4-Amino-1-(2-pyridyl)-piperidin der Formel 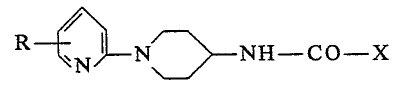 worin R und X obige Bedeutung haben, unter sauren oder basischen Bedingungen hydrolysiert und das Endprodukt isoliert und/oder in seine pharmazeutisch akzeptablen Salze übergeführt wird.

10. Verfahren nach Anspruch 9, dadurch gekennzeichnet, daß als geschütztes 4-Aminopiperidin 4-Acetamidopiperidin verwendet wird.

11. Medikament, welches als Wirkstoff ein Produkt gemäß den Ansprüchen 1 bis 8 enthält.

12. Medikament nach Anspruch 11 in Form eines pharmazeutischen Präparates mit einem Gehalt von 0,5 bis 250 mg Wirkstoff pro Dosiseinheit.

13. Medikament nach den Ansprüchen 11 und 12, dadurch gekennzeichnet, daß es für orale Verabreichung formuliert ist.

## Claims

1. 4-amino-1-(2-pyridyl)piperidines of general formula: 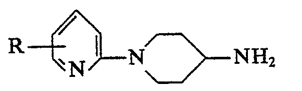 in which R represents hydrogen, a halogen, a methyl group, a trifluoromethyl group, a lower alkoxy group, a trifluoromethyl group, a 2,2,2-trifluoroethoxy group, a lower alkylthio group, a trifluoromethylthio group, a phenoxy group, a phenoxy group substituted in the phenyl ring by a halogen, a trifluoromethyl group, a lower alkyl, a lower alkoxy, a lower alkylthio or a cyano group, a phenylthio group or a phenylthio group substituted in the phenyl ring by a halogen, a trifluoromethyl group, a lower alkyl, a lower alkoxy, a lower alkylthio or a cyano group, and pharmaceutically acceptable salts thereof, the terms »lower alkyl«, slower alkoxyt and »lower alkythioc designating groups containing the residue of a saturated aliphatic hydrocarbon containing 1 to 3 atoms of carbon.

2. 4-amina-1-(6-chloro-2-pyridyl)piperidine and pharmaceutically acceptable salts thereof.

3. Monohydrochloride of 4-amino-1-(6-chloro-2-pyridyl)-piperidine.

4. Dihydrochlorideof4-amino-1-[6-(2,2,2-trifluoro)ethoxy-2-pyridyl)piperidine.

5. Dihydrochloride of 4-amino-1-(6-methy)-2-pyridyl)piperidine.

6. Monohydrochtorideof4-amino-1-(6-bromo-2-pyridyl)piperidine.

7. Monohydrochloride of 4-amino-1 -(6-phenylthio-2-pyridyl)piperidine.

8. Monohydrochloride of 4-amino-1-(6-methoxy-2-pyridy))piperidine.

9. Process for preparing 4-amino-1-(2-pyridyl)piperidines of formula 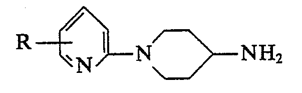 in which R represents hydrogen, a halogen, a methyl group, a trifluoromethyl group, a lower alkoxy group, a trifluoromethoxy group, a 2,2,2-trifluoroethoxy, a lower alkylthio group, a trifluoromethylthio group, a phenoxy group, a phenoxy group substituted in the phenyl ring by a halogen, a trifluoromethyl group, a lower alkyl, a lower alkoxy, a lower alkylthio or a cyano group, a phenylthio group, or a phenylthio group substituted in the phenyl ring by a halogen, a trifluoromethyl group, a lower alkyl, a lower alkoxy, a lower alkylthio or a cyano group, and for preparing the pharmaceutically acceptable salts thereof, characterized in that a 2-halopyridine of formula 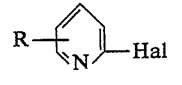 in which R has the above-given significance and Hal represents an atom of halogen is treated with a blocked 4-aminopiperidine of formula 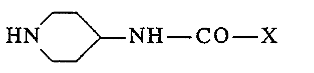 in which X represents an atom of hydrogen or a lower alkyl group, the N-substituted 4-amino-1 -(2-pyridyi)piperidine thus obtained of formula 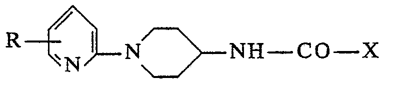 in which R and X have the significance given above, is hydrolysed under acid or basic conditions, and the final product is isolated and/or converted into its pharmaceutically acceptable salts.

10. A process according to claim 9, characterized in that 4-acetamidopiperidine is used as blocked 4-aminopiperidine.

11. A drug containing as active ingredient a product according to claims 1 to 8.

12. A drug according to claim 11 in the form of a pharmaceutical composition containing 0.5 to 250 mg of active ingredient par dosage unit.

13. A drug according to claim 11 and 12, characterized in that it is formulated for oral administration.
